# EUROPEAN PATENT SPECIFICATION

(11) **EP 2 123 266 B1**
(45) Date of publication and mention of the grant of the patent: **15.01.2014**
(21) Application number: 07850213.5
(22) Date of filing: 06.12.2007
(51) Int. Cl.: A61K 31/09, A61P 35/00, A61P 43/00

(54) **CANCER THERAPEUTIC AGENT AND ANTI-CARCINOGENIC AGENT**
THERAPEUTISCHES KREBSMITTEL UND ANTI-KARZINOGENES MITTEL
AGENT THÉRAPEUTIQUE CONTRE LE CANCER ET AGENT ANTI-CARCINOGÈNE

(30) Priority: 06.12.2006 JP 2006329556; 26.07.2007 US 935104 P
(43) Date of publication of application: 25.11.2009
(73) Proprietor: KANEKA CORPORATION, Osaka (JP)
(72) Inventor: FUJII, Toshihide, Kobe-shi Hyogo 655-0872 (JP); KATO, Masanori, Akashi-shi Hyogo 674-0057 (JP); SAKAMOTO, Hirokazu, Kobe-shi Hyogo 657-0845 (JP); SHINAGAWA, Yoshiyuki, Akashi-shi Hyogo 674-0051 (JP); KITANO, Mitsuaki, Takasago-shi Hyogo 676-0013 (JP); HOSOE, Kazunori, Takasago-shi Hyogo 676-0025 (JP)
(74) Representative: Gillet, Raphaëlle
(86) International application number: PCT/JP2007/073604
(87) International publication number: WO 2008/069276

(56) References cited:
- EP-A- 1 870 095
- EP-A- 2 005 951
- WO-A-2006/104153
- WO-A-2006/104154
- WO-A1-03/032968
- WO-A1-03/061395
- WO-A1-2006/104153
- JP-A- 07 196 534
- VOLLING P ET AL: "[Adverse effects of cytostatic chemotherapy in patients with head and neck carcinomas. Prevention and therapy]" HNO NOV 1999, vol. 47, no. 11, November 1999 (1999-11), pages 999-1015, XP002556745 ISSN: 0017-6192
- YAMAMOTO Y.: 'Seitai-nai Kosanka Busshitsu no Yakuwari to Sono Atarashii Kangaekata' SOCIETY SANKA SEMINAR 05 December 1997, pages 15 - 19
- NOHL H. ET AL.: 'Ubiquinol and papaverine derivative caroverine prevent the expression of tumour-promoting factors in adenoma and carcinoma colon cancer cells induced by dietary fat' BIOFACTORS vol. 25, 2005, pages 87 - 95, XP008108967

## Description

### Technical Field

The present invention relates to an agent for use in a method for mitigating adverse reactions of anticancer agents, which can be used in foods and beverages, such as health foods (supplements, health aid foods, nutritional supplementary foods, nutrient-fortified foods, nutrient-adjusted foods, health beverages) and health or nutrition functional foods (foods for specified health uses, foods with nutrient function claims), or in pharmaceuticals, cosmeceutical, cosmetics.

### Background of the Invention

A broad range of studies have been conducted to date concerning methods for suppressing carcinogenesis or for treating cancer. From the viewpoint of suppressing the onset of cancer, emphasis is placed on the exploration of carcinogenesis-suppressing substances. This occupies an important position also for the purpose of suppressing the recurrence of cancer in patients who have undergone surgery to extirpate cancer mass. If an effective carcinogenesis-suppressing substance, whether extracted or synthesized, can be used to suppress carcinogenesis in the form of pharmaceuticals, foods and the like containing the substance, not only the prevention of cancer recurrences, but also a suppressive effect on carcinogenesis is expected in cancers that are highly likely to occur, particularly such as hereditary or occupational cancers. Such pharmaceuticals, foods and the like are thought to make major contributions to emerging new medicine and the maintenance of health in daily life.

A great deal of research and product development has been undertaken for cancer therapeutic agents, many of which are already in actual use in medical settings. However, no drugs capable of radical treatment of cancer have been developed. Problems with currently available cancer therapeutic agents, including anticancer agents and immunotherapeutic agents, arise from difficulty in allowing the drug to act selectively on cancer cells without affecting normal cells, and from the safety issue involved intrinsically in chemically synthesized drugs. As a result, effective doses (minimum effective concentrations) and doses allowable from safety (minimum toxic concentrations) are very close to each other, or minimum toxic concentrations are lower than minimum effective concentrations; no sufficient efficacy has been obtained. Against this background, there is a demand for the development of widely applicable superior anticancer agents with low prevalence of adverse reactions.

Although administration of anticancer agents is essential in cancer treatment, severe adverse reactions, such as anorexia, general malaise, pain, sensation of difficulty in respiration, skin symptoms, nausea and vomiting, diarrhea, fever, hair loss, anosmia, hepatic or renal dysfunction, interstitial pneumonia, organ failure due to peripheral circulatory failure, and bone marrow suppression, are unavoidable for the reasons described above. In recent years, a mainstream concept for cancer treatment has been to think that improving the quality of life (QOL) for cancer patients is also an important effect of treatment, as well as the extension of their survival. How to mitigate the adverse reactions of anticancer agents represents an important problem. If the dosage of anticancer agents for patients on cancer treatment can be reduced, the adverse reactions of the anticancer agents will be mitigated, and cancer patients will be freed from both physical and mental sufferings.

It is preferable that a therapeutic agent and carcinogenesis suppressing agent for the prevention of recurrences be conveniently ingestible particularly in daily life. Most importantly, long-time administration is likely; the agents are desirably a highly safe substance and drug with low prevalence of adverse reactions to the body. Unfortunately, however, no carcinogenesis suppressing agent or cancer therapeutic agent with high utility and high safety that meet these requirements have been found to date.

Coenzyme Q is a substance that is essential for the maintenance of the functions of living organisms, known to be localized in mitochondria, lysosome, Golgi apparatus, microsome, peroxysome, cell membrane and the like, and to be involved as a component of the mitochondrial electron transfer system in adenosine triphosphate (ATP) production activation, antioxidant action in vivo, and membrane stabilization. The coenzyme Q in vivo mostly occurs in reduced form; once absorbed in the body, oxidized coenzyme Q is converted to the reduced form by reductase in the cells.

Oxidized coenzyme Q is a compound widely utilized as a therapeutic drug for congestive heart failure and as a supplement; its utility has been reported not only in heart function, but also in a broad range of diseases, including arteriosclerosis, hypertension, diabetes mellitus, and brain disease, and its adverse reactions have been reported infrequently. For example, in a safety study of oxidized coenzyme Q₁₀ in rats, no toxic signs were observed with administration at a high dose of 1.2 g/kg/day for 52 consecutive weeks, demonstrating high safety.

Likewise, reduced coenzyme Q has been shown to be highly safe.

Regarding oxidized coenzyme Q, some reports are available on its therapeutic effects on cancers including breast cancer, bronchogenic cancer and rectal cancer (JP-A-2003-135022; JP-T-2003-532679
and Biochem. Biophys. Res. Commun. (1994)199, 1504-1508 ; BioFactors (1999)9, 365-370 ; Biochem. Biophys. Res. Commun. (1993)192, 241-245). Recently, a report was presented on carcinogenesis suppression by oxidized coenzyme Q (WO 2007/013556).

A patent document is available stating that preparations containing an antioxidant are effective in the treatment of a wide variety of diseases, including tumorigenesis (WO 2002/034303), wherein ubiquinone (oxidized coenzyme Q) or ubiquinol (reduced coenzyme Q) is disclosed among the many examples of antioxidant components mentioned therein, and tumorigenesis is only one among the many diseases mentioned therein.

LDL (low-density lipoprotein), a kind of lipoprotein wherein a lipid and apoprotein are bound, has been previously shown to have a cell proliferation effect in fibroblasts (Am. J. Physiol. (2003)284, H644-H653) or vascular endothelial cells (Cardiovasc. Res. (2003) 59, 934-944); intracellular signaling is known to be a factor involved in the mechanism for the cell proliferation.
Signal proteins such as cyclin D1, cyclin B1, p53, and p21, have been shown to be expressed in the intracellular signaling, and are also known to be profoundly associated with cancer cell proliferation and the cell proliferation induced in the carcinogenesis process.
WO2006/104154 discloses a composition containing coenzyme Q as an active constituent for enhancing expression and activity of a protease in the liver.
WO2006/104153 (EP 1 870 095) discloses a composition comprising reduced coenzyme Q as the active ingredient for increasing the anti-oxidation activity in the blood in an elderly mammal.
Volling P. and Ebeling O., 1999, HNO, 47 :999-1015 is a review which summarizes different data (pharmacology, dosage, side effects etc) concerning several anti-cancer drugs.

### Disclosure of the Invention

### Problems to be Solved by the Invention

An object of the present invention is to provide an agent for mitigating adverse reactions of anticancer agents.

### Means of Solving the Problems

Surprisingly, the present inventors found that reduced coenzyme Q solved the aforementioned problems.
Accordingly, the present invention provides the following.
[1]An agent for use in a method for mitigating adverse reactions of anticancer agents, said agent comprising a reduced coenzyme Q represented by the formula (1): 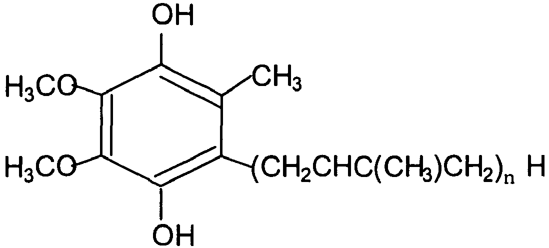 (wherein n represents an integer of 1 to 12) as an active ingredient.
   [2] The agent according to [1] above, wherein the reduced coenzyme Q is reduced coenzyme Q₁₀.
   [3] The agent according to [1] above, comprising 0.001 to 99.9% by weight of the reduced coenzyme Q.
   [4] The agent according to any one of [1] to [3] above, wherein the daily dose of the reduced coenzyme Q is 1 mg to 3000 mg.
   [5] The agent according to any one of [1] to [4] above, which is a pharmaceutical, a cosmeceutical or a cosmetic.
   [6] The agent according to any one of [1] to [4] above, which is a food or a beverage.
   [7] The agent according to any one of [1] to [4] above, which is an animal drug, an animal feed or an animal food.

### Effect of the Invention

The present invention provides an agent utilizing reduced coenzyme Q, for use in a method for mitigating the adverse reactions of an anticancer agent. Such an agent can be utilized in foods and beverages, including general foods, health foods (supplements, health aid foods, nutritional supplementary foods, nutrient-fortified foods, nutrient-adjusted foods, health beverages), and health or nutrition functional foods (foods for specified health uses, foods with nutrient function claims), or pharmaceuticals, cosmeceuticals, and cosmetics. In particular, for cancer patients undergoing treatment with an anticancer agent that involves severe adverse reactions, such as pain and depression, a safer cancer therapeutic agent with reduced prevalence of adverse reactions can be provided by combining a reduced coenzyme Q and the anticancer agent. The present invention provides an agent for use in a method for mitigating the adverse reactions of an anticancer agent that is not a reduced coenzyme Q by using a reduced coenzyme Q in combination with the anticancer agent.

### Brief Description of the Drawings

Fig. 1 is a graph showing the suppressive effect of reduced coenzyme Q₁₀ on LDL-induced cell proliferation in human skin fibroblasts.
Fig. 2 is a graph showing suppressive effects of oxidized coenzyme Q₁₀ and reduced coenzyme Q₁₀ on cell activation in cancer-derived cells.

### Best Mode for Carrying out the Invention

Embodiments of the present invention are hereinafter described in detail.

The above-described agent for use in a method for mitigating adverse reactions of anticancer agents (hereinafter also generically referred to as "the agent of the present invention") is a composition comprising a reduced coenzyme Q as an active ingredient. As stated above, there are two forms of coenzyme Q: oxidized and reduced, which are represented respectively by the formula (1) :

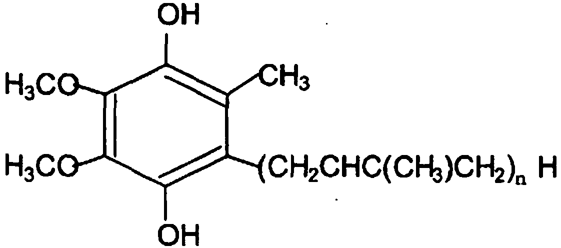

(wherein n represents an integer of 1 to 12), or the formula (2):

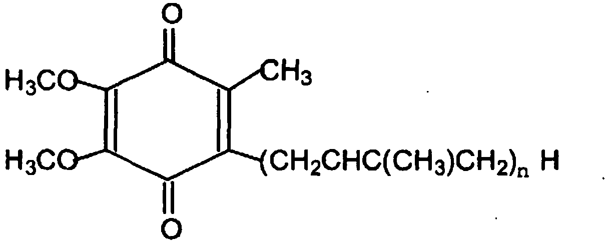

(wherein n represents an integer of 1 to 12). The formula (1) represents a reduced coenzyme Q; the formula (2) represents an oxidized coenzyme Q.

In the agent of the present invention, a reduced coenzyme Q, out of the above-described coenzyme Q species, serves as an active ingredient; however, as long as the utility such as efficacy of the reduced coenzyme Q and the effect of the present invention are not adversely affected, an oxidized coenzyme Q may be present with the reduced coenzyme Q. When a mixture of reduced coenzyme Q and oxidized coenzyme Q is used as the coenzyme Q, it is preferable, from the viewpoint of maximizing the effect of the present invention, that the ratio of the reduced coenzyme Q be not less than 60% by weight, more preferably not less than 80% by weight, most preferably not less than 95% by weight, relative to the total coenzyme Q. When a mixture of reduced coenzyme Q and oxidized coenzyme Q is used as the coenzyme Q, the content of the reduced coenzyme Q in the mixture is preferably as high as possible, and the upper limit of the content in the mixture is, for example, 99.5% by weight. Of course, it is also preferable to use the reduced coenzyme Q alone.

A reduced coenzyme Q as an active ingredient of the agent of the present invention, or a coenzyme Q as a mixture of a reduced coenzyme Q and an oxidized coenzyme Q, can be obtained by any method. Useful methods include, but not limited to, for example, a method comprising obtaining a coenzyme Q based mainly on the oxidized type by a commonly known conventional method such as fermentation, chemical synthesis, or extraction from a natural substance, and then concentrating the reduced coenzyme Q fraction in the effluent by chromatography as required. A reduced coenzyme Q can be obtained by adding an ordinary reducing agent such as sodium borohydride or sodium dithionite (hydrosulfite sodium) to the above-described coenzyme Q as required, and reducing the oxidized coenzyme in the above-described coenzyme Q by a conventional method to yield a reduced coenzyme Q, which may then be concentrated by chromatography. A reduced coenzyme Q can also be obtained by reacting an existing high-purity oxidized coenzyme Q with the aforementioned reducing agent(s).

Examples of the reduced coenzyme Q as an active ingredient of the agent of the present invention or the oxidized coenzyme Q that can be used as a component of a mixture with the reduced coenzyme Q include those having 1 to 12 side chain repeat units (in the formulas, n) as represented by the formulas (1) and (2) above. In particular, because one having 10 side chain repeat units, i.e., coenzyme Q₁₀, is the primary component of in vivo coenzyme Q in humans, as well as in dogs and many other companion animals (e.g., monkeys, pigs, cats, horses, sheep, rabbits, guinea pigs, chicken, turkeys), reduced coenzyme Q₁₀ and oxidized coenzyme Q₁₀ can be particularly suitably used from the viewpoint of safety and efficacy when administered to humans, dogs, monkeys, pigs, cats, horses, sheep, rabbits, guinea pigs, chicken, turkeys and other subjects of administration.

Cancer is a disease characterized by onset or progression due to abnormal cell proliferation; the cell proliferation induced by a broad range of environmental, chemical and genetic factors is recognized as an important biochemical indicator in the promotion stage of carcinogenesis and the progression of cancer. As described in Examples below, reduced coenzyme Q suppressed the abnormal cell proliferation induced by the addition of LDL, a chemical factor, in otherwise normally functioning cells. It can be concluded, therefore, that reduced coenzyme Q is effective as a carcinogenesis suppressing agent.

As used herein, the term "carcinogenesis suppression" means the suppression of the occurrence of cancer and even the prevention of cancer, by suppressing the genesis and proliferation of cancer cells. The carcinogenesis suppressing agents can be used for the purpose of, for example, suppressing carcinogenesis in hereditary cancers, suppressing carcinogenesis in cancers associated with the environment or lifestyles, and suppressing and even preventing the recurrence following tumor extirpation surgery and metastasis of cancer.

Although transformed cells are capable of infinite proliferation, cell activation is essential in the stage preceding the proliferation. As can be seen in Examples below, reduced coenzyme Q activates normal cells; however, it was found that reduced coenzyme Q has a cell activation suppressive effect only on transformed cells showing abnormal proliferation. When the activation of cancer cells is suppressed, their proliferation is also suppressed. Therefore, reduced coenzyme Q is effective as a cancer cell activation suppressing agent and hence as a cancer therapeutic agent. As used herein, the term suppression of cancer cell activation refers to a reduction in the ATP content in cancer cells.

As used herein, the term "cancer treatment" means, but is not limited to, suppression of cancer metastasis, suppression of cancer progression, cancer shrinkage and the like, as well as suppression of the proliferation of once-generated cancer cells. The agent of the present invention can be used as part of cancer therapy having the above-described purposes of suppressing the proliferation of cancer cells, suppressing cancer metastasis, suppressing cancer progression, shrinking cancer masses.

Because of the above-described cancer therapeutic effects, reduced coenzyme Q is able to reduce the dosage of other anticancer agents, and hence to mitigate the adverse reactions of the anticancer agents when used in combination with the anticancer agents. Such combination drugs are capable of mitigating the adverse reactions of other anticancer agents. Therefore, reduced coenzyme Q is capable of mitigating the adverse reactions of other anticancer agents.

As used herein, the term "mitigation of the adverse reactions of anticancer agents" means mitigation of adverse reactions due to administration of existing anticancer agents, a practice essential in cancer treatment settings, and also means mitigation of adverse reactions due to administration of anticancer agents that will possibly be developed in the future.

The choice of tumor targeted by the agent of the present invention is not limited. That is, the tumor may be an epithelial tumor or a non-epithelial tumor, whether benign or malignant. Both primary tumors and metastatic or recurrent tumors are included. The tumor can be a solid tumor or a tumor of the blood or hematopoietic system. For example, the tumor may be any of tumors such as epithelial tumors and non-epithelial tumors that develop in various organs, including the stomach, small intestine, large intestine, rectum, colon, lung, liver, kidney, pancreas, gall bladder, uterus, ovary, testis, prostate, skin and brain, nervous system tumors, bone tumors, and lymph system tumors. More specifically, for example, breast cancers (e.g., infiltrative mammary duct cancer, non-infiltrative mammary duct cancer, inflammatory breast cancer), prostatic cancers (e.g., hormone-dependent prostatic cancer, non-hormone-dependent prostatic cancer), pancreatic cancers (e.g., pancreatic duct cancer), gastric cancers (e.g., papillary adenocarcinoma, mucinous adenocarcinoma, adenosquamous carcinoma), lung cancers (e.g., non-small cell lung cancer, small cell lung cancer, malignant mesothelioma), colic cancers (e.g., gastrointestinal interstitial tumor), rectal cancers (e.g., gastrointestinal interstitial tumor), colorectal cancers (e.g., familial colorectal cancer, hereditary non-polyposis colorectal cancer, gastrointestinal interstitial tumor), small intestine cancers (e.g., non-Hodgkin's lymphoma, gastrointestinal interstitial tumor), esophageal cancer, duodenal cancer, cancer of tongue, pharyngeal cancers (e.g., nasopharyngeal cancer, oropharyngeal cancer, hypopharyngeal cancer), salivary gland cancer, brain tumors (e.g., pineal astrocytoma, hairy cell astrocytoma, diffuse astrocytoma, anaplastic astrocytoma), schwannoma, liver cancers (e.g., primary liver cancer, extrahepatic bile duct cancer), renal cancers (e.g., renal cell carcinoma, transitional epithelium cancer of renal pelvis and ureter), bile duct cancer, endometrial cancer, cervical cancer, ovarian cancers (e.g., epithelial ovarian cancer, extragonadal germ cell tumor, ovarian germ cell tumor, low-malignancy ovarian tumor), bladder carcinoma, urethral cancer, skin cancers (e.g., intraocular (ocular) melanoma, Merkel cell cancer), angioma, malignant lymphoma, malignant melanoma, thyroid cancers (e.g., thyroid medullary carcinoma), parathyroid cancer, nasal sinus cancer, paranasal sinus cancer, bone tumors (e.g., osteosarcoma, Ewing's sarcoma, uterine sarcoma, soft tissue sarcoma), angiofibroma, retinal sarcoma, penile cancer, testicular tumor, pediatric solid cancers (e.g., Wilms tumor, pediatric renal tumor), Capodi's sarcoma, AIDS-related Capodi's sarcoma, maxillary sinus tumor, fibrous histiocytoma, smooth muscle sarcoma, rhabdomyosarcoma, leukemias (e.g., acute myelocytic leukemia, acute lymphoblastic leukemia) and the like can be mentioned, with colorectal cancers, breast cancers, liver cancers, prostatic cancers, leukemias and the like being preferable, and colorectal cancers and breast cancers being more preferable.

Because the agent of the present invention comprises a reduced coenzyme Q which is free from the fear of adverse reactions as an active ingredient thereof, it can be used directly as an agent for mitigating the adverse reactions of cancer, and hence can be used for the purpose of improving the QOL for patients on cancer treatment. As used herein, the term "improving the QOL for patients on cancer treatment" includes mitigating the physical sufferings of cancer patients during and after cancer treatment to thereby satisfy the cancer patients mentally. More specifically, the term refers to mitigating physical disorders and discomforts such as anorexia, general malaise, pain, sensation of difficulty in respiration, skin symptoms, nausea/vomiting, diarrhea, fever, hair loss, anosmia, hepatic or renal dysfunction, interstitial pneumonia, organ failure due to peripheral circulatory failure, and bone marrow suppression as adverse reaction symptoms resulting from administration of anticancer agents, to thereby free the patients from mental sufferings such as anxiety, agitation, loss of interest, emotional paralysis, insomnia, feeling of alienation, fear, maladjustment, depression, and delirium.

Regarding the timing of use, the agent of the present invention can be used not only during cancer treatment, but also during administration of anticancer agents used for prevention of recurrences after cancer treatment, and even after discontinuation of the anticancer agents. Specific examples of use of the agent of the present invention include, but are not limited to, a case wherein a person recognized by genetic diagnosis as being likely to experience carcinogenesis in the future takes the agent of the present invention before onset so as to suppress or prevent carcinogenesis, a case wherein a person who realizes the likelihood of familial carcinogenesis takes the agent of the present invention so as to suppress or prevent carcinogenesis, a case wherein the agent of the present invention is administered in place of, or concurrently with, an anticancer agent for chemotherapy, a case wherein a person who suffers considerable deterioration of QOL due to adverse reactions of administration of anticancer agents takes the agent of the present invention in terminal care with no expectations for healing, and a case wherein the dosage of existing anticancer agents or anticancer agents that can be developed in the future is reduced by using them in combination with the agent of the present invention, to thereby mitigate the adverse reactions resulting from the anticancer agents.

Existing anticancer agents that can be used in combination with the agent of the present invention include, but are not limited to, aminoglutethimide, azacitidine, amsacrine, anastrozole, asparaginase, ancitabine, altretamine, idarubicin, irinotecan, ifosfamide, imatinib, interferon-α, uracil mustard, epirubicin, estramustine, etoposide, exemestane, enocitabine, octoreotide, oxaliplatin, capecitabine, carboplatin, carmustine, carmofur, cladribine, clodronate, chlorambucil, gemcitabine, goserelin, cisplatin, cytarabine, cyclophosphamide, cyproterone, diethylstilbestrol, streptozocin, dactinomycin, daunomycin, daunorubicin, tamoxifen, thiotepa, 6-thioguanine, tyrphostin, dacarbazine, tegafur, teniposide, docetaxel, doxorubicin, topotecan, trastuzumab, tretinoin, doxifluridine, toremifene, nilutamide, nimustine, nedaplatin, nogitecan, paclitaxel, pamidronate, pantostatin, vindesine, vinorelbine, vinblastin, vincristine, bicalutamide, hydroxyurea, hydroxycarbamide, pirarubicin, fluorouracil, fludrocortisone, flutamide, fluoxymesterone, fludarabine, plicamycin, procarbazine, bleomycin, buserelin, busulfan, fadrozole, peplomycin, pentostain, porfimer, mitomycin C, mitotane, mitoxantrone, melphalan, methotrexate, mercaptopurine, mechlorethamine, raltitrexed, ranimustine, rituximab, leuprorelin, letrozole, Leucovorin, leuprolide, lomustine and the like. As used herein, the term "used in combination" includes preparing a mixture of a reduced coenzyme Q and an existing anticancer agent for a single agent, or preparing both separately and taking them simultaneously or at a time interval.

The dosage of the agent of the present invention is variable depending on target disease, subject of administration, symptoms, presence or absence of its combination with the existing anticancer agent(s), route of administration, and the like, but is not limited; based on the amount of reduced coenzyme Q, the daily dose for each adult human (weighing 60 kg) is 1 mg/day to 3000 mg/day (17 µg/kg/day to 50 mg/kg/day), preferably 30 mg/day to 600 mg/day (500 µg/kg/day to 10 mg/kg/day), more preferably 60 mg/day to 300 mg/day (1 mg/kg/day to 5 mg/kg/day). If the reduced coenzyme Q is used in non-human animals (dogs assumed), the daily dose for each mature animal is 830 µg/kg/day to 2.5g/kg/day, preferably 25 mg/kg/day to 500 mg/kg/day, more preferably 50 mg/kg/day to 250 mg/kg/day.

The agent of the present invention can be used as pharmaceuticals, cosmeceuticals, cosmetics, foods such as functional foods (supplements, health aid foods, nutritional supplementary foods, nutrient-fortified foods, nutrient-adjusted foods, health beverages, foods for specified health uses, foods with nutrient function claims), animal drugs, and animal feeds or foods (pet foods, livestock feeds and the like). The agent of the present invention contains a coenzyme Q in an amount appropriate for the use of the above-described dosage to the subject, depending on the intended use thereof. The agent of the present invention may be taken in a single daily dose described above, and may be taken in several divided portions.

For example, when the food of the present invention is what is called a health food, a form wherein not less than 100 µg, preferably not less than 1 mg, more preferably not less than 10 mg, of a powder or granular reduced coenzyme Q is packaged in a unit serving form. When the food is a health drink, a form wherein not less than 100 µg, preferably not less than 1 mg, more preferably not less than 10 mg, of a reduced coenzyme Q is suspended or dissolved in a drink and contained in a bottle or the like for a single consumption can be mentioned. In the case of these forms, the upper limit of the amount of reduced coenzyme Q is preferably 300 mg per serving. As used herein, "a unit serving form" refers to a form for which the amount to be taken per serving is defined in advance; for example, in the case of beverages, candies, chewing gums, jellies, puddings, yogurt, a given amount can be defined by packs, packages, bottles; in the case of granular, powder, or slurry foods, a given amount can be defined by packages, or the amount to be taken per serving may be indicated on containers.

For the above-described uses, the agent of the present invention may be formulated with other pharmaceutically and food-hygienically acceptable materials which may be added and mixed as appropriate by a conventional method with the reduced coenzyme Q. Examples of such materials include, but are not limited to, an excipient, a disintegrant, a lubricant, a binder, a coating agent, a colorant, an antiflocculant, an absorption promoter, a solubilizer, a stabilizer, an isotonizing agent, a health food material, a nutritional supplementary food material, a vitamin, a flavor, a sweetening agent, an antiseptic, an antioxidant.

Examples of the excipient include, but are not limited to, white soft sugar, lactose, glucose, cornstarch, mannitol, crystalline cellulose, calcium phosphate, and calcium sulfate.

Examples of the disintegrant include, but are not limited to, starch, agar, calcium citrate, calcium carbonate, sodium hydrogen carbonate, dextrin, crystalline (/microcrystalline) cellulose, carboxymethylcellulose, and tragacanth.

Examples of the lubricant include, but are not limited to, talc, magnesium stearate, polyethylene glycol, silica, and hardened vegetable oils.

Examples of the binder include, but are not limited to, ethylcellulose, methylcellulose, hydroxypropylmethylcellulose, tragacanth, shellac, gelatin, gum arabic, polyvinylpyrrolidone, polyvinylalcohol, polyacrylic acid, polymethacrylic acid, and sorbitol.

Examples of the coating agent include, but are not limited to, gum arabic, Opadry, self-heal (Prunella vulgaris), castor wax, carboxyvinyl polymer, carmellose, hydrated silicon dioxide, magnesium silicate, vinyl acetate resin, stearic acid, cetanol, and hydroxypropylmethylcellulose.

Examples of the colorant include, but are not limited to, colorants approved for use in pharmaceuticals or foods.

Examples of the antiflocculant include, but are not limited to, stearic acid, talc, light silicic anhydride, and hydrated silicon dioxide.

Examples of the absorption promoter include, but are not limited to, surfactants such as higher alcohols (for example stearyl alcohol), higher fatty acids, and glycerin fatty acid esters (polyoxyethylene glycerol sorbitan fatty acid esters, polyoxyethylene fatty acid esters).

Examples of the solubilizer include, but are not limited to, organic acids such as fumaric acid, succinic acid, and malic acid.

Examples of the stabilizer include, but are not limited to, benzoic acid, sodium benzoate, ethyl para-oxybenzoate, and magnesium sulfate.

Examples of the isotonizing agent include, but are not limited to, calcium chloride.

Examples of the health food materials include, but are not limited to kanpo medicines (e.g., ireito, unkeito, unsei'in, ogi-kentyuto, oren-gedokuto, orento, kakkonto, kami-kihito, kami-syoyosan, kanbaku-daisoto, kikyoto, kihito, kyumi-binroto, keigai-rengyoto, keisi-ka-syakuyakudaioto, keihi-ka-syakuyakuto, keihi-ka-ryukotu-boreito, keisito, keisi-ninzinto, keisi-bukuryogan, keihito, kososan, gokoto, gosyakusan, gosha-jinkigan, gorinsan, saikanto, saiko-ka-ryukotu-boreito, saiko-keisi-kankyoto, saikokeisito, saiko-seikanto, saibokuto, saireito, sansoninto, ziin-kokato, sigyakusan, sikunsito, simotuto, sya-kanzoto, syakuyakukanzoto, zyuzen-taihoto, zyumi-haidokuto, syokentyuto, syosaikoto, syoseiryuto, syohusan, sin'i-seihaito, sinpito, sinbuto, seizyo-bohuto, seisyo-ekkito, seisinrensiin, seihaito, sokei-kakketuto, daio-kanzoto, daiobotanpito, daikentyuto, daisaikoto, daisaikoto-kyo-daio, daijyokito, daibohuto, jidaboku-ippo, tyoi-zyokito, tyotosan, tyoyoto, tyoreito, tyoreito-go-simotuto, tudosan, tokaku-zyokito, toki-insi, toki-kentyuto, toki-syakuyakusan, tokito, nitinto, nyosinsan, ninzinto, ninzin-yoeito, hainosankyuto, bakumondoto, hatimi-ziogan, hange-kobokuto, hange-syasinto, byakko-ka-ninzinto, bukuryoin, bukuryoingo-hange-kobokuto, heiisan, boi-ogito, bohu-tusyosan, hotyu-ekkito, maoto, mao-busi-saisinto, makyo-kansekito, masiningan, mokuboito, yokukansan, yokukansan-ka-tinpihange, rikkunsito, rikkosan, ryutan-syakanto, ryokankyo-misingeninto, rokumi-ziogan), tea leaves (e.g., green tea, unmilled rice tea, powdered tea, green tea of middle grade, toasted tea, roasted tea, jasmine tea, oolong tea, hongcha, heicha, huacha, jincha, baicha), herbs (e.g., Italian parsley, elecampane, olive, oregano, cardoon, chamomile, curry plant, catnip, caraway, Christmas rose, crimson clover, cornflower, common mallow, salad burnet, santolina, cinnamon, jasmine, stevia, sage, European linden, scented geranium, St.-John's-wort, soapwort, Solomon's-seal, thyme, tansy, chervil, chive, nasturtium, jujube, basil, honeysuckle, hyssop, flax, fennel, foxglove, black hollyhock, French marigold, betony, heliotrope, bergamot, hemp agrimony, rue, pot marigold, borage, white horehound, myrtle, mullein, marjoram, mint, yarrow, lavender, lady's bedstraw, lemon grass, lemon verbena, lemon balm, rose, rosemary, rocket, wild strawberry, wild pansy, forget-menot), propolis, gingko leaf, aojiru (green-leaved-vegetable juice) and extract thereof.

Examples of the nutritional supplementary food materials include, but are not limited to, amino acids, metal ions, proteins, saccharides, fatty acids, yeast extract, vegetable extract, fish meat extract, fruit, fruit extract.

Examples of the vitamin include, but are not limited to, for example, vitamin A, vitamin B, vitamin C, vitamin D, vitamin E, and vitamin K.

Examples of the flavor include, but are not limited to, single-ingredient flavors such as menthol, carvone, anethole, cineole, methyl salicylate, cinnamic aldehyde, eugenol, 3,1-menthoxypropane-1,2-diol, thymol, linalol, linalyl acetate, limonene, menthone, menthyl acetate, N-subsituted-para-menthane-3-carboxamide, pinene, octylaldehyde, citral, pulegone, carvyl acetate, anise aldehyde, ethyl acetate, ethyl butyrate, arylcyclohexane propionate, methyl anthranylate, ethylmethylethinyl glycidate, vanillin, undecalactone, hexanal, ethyl alcohol, propyl alcohol, butanol, isoamyl alcohol, hexenol, dimethyl sulfide, cyclotene, furfural, trimethylpyrazone, ethyl lactate, and ethyl thioacetate; as well as natural flavors such as peppermint oil, spearmint oil, anise oil, eucalyptus oil, wintergreen oil, cassia oil, clove oil, thyme oil, sage oil, lemon oil, orange oil, mentha oil, cardamom oil, coriander oil, mandarin oil, lime oil, lavender oil, rosemary oil, laurel oil, chamomile oil, caraway oil, marjoram oil, bay oil, lemon-grass oil, origanum oil, pine needle oil, neroli oil, rose oil, jasmine oil, iris concrete, absolute peppermint, absolute rose, and orange flower; blended flavors such as strawberry flavor, apple flavor, banana flavor, pineapple flavor, grape flavor, mango flavor, butter flavor, milk flavor, fruit mix flavor, and tropical fruit flavor.

Examples of the sweetener include, but are not limited to, saccharin sodium, aspartame, stevioside, stevia extract, para-methoxycinnamic aldehyde, neohesperidyl dihydrochalcone, perilla rutin.

Examples of the antiseptic include, but are not limited to, aminoethylsulfonic acid, benzoic acid, sodium benzoate, sodium edetate, agar, dl-camphor, citric acid, sodium citrate, salicylic acid, sodium salicylate, phenyl salicylate, dibutylhydroxytoluene, sorbic acid, potassium sorbate, nitrogen, dehydroacetic acid, sodium dehydroacetate, 2-naphthol, white soft sugar, honey, parabens (for example, isobutyl para-oxybenzoate, isopropyl para-oxybenzoate, ethyl para-oxybenzoate, butyl para-oxybenzoate, propyl para-oxybenzoate, methyl para-oxybenzoate), 1-menthol, eucalyptus oil.

Examples of the antioxidant include, but are not limited to, citric acid, vitamin C and the derivative ascorbyl stearate, lycopene, vitamin A, carotenoids, vitamin B, flavonoids, polyphenols, glutathione, dibutylhydroxytoluene, butylhydroxyanisole, gallic acid esters, selenium, sodium thiosulfate, vitamin E, α lipoic acid, pycnogenol, flavangenol, astaxanthin, catechins, pyrroloquinolinequinone, coenzyme A, superoxide dismutase (SOD), glutathione peroxidase, glutathione-S-transferase, glutathione reductase, catalases, ascorbic acid peroxidase, and mixtures thereof. In particular, antioxidants are used as additives particularly preferably from the viewpoint of increasing the oxidation stability of the reduced coenzyme Q, and hence obtaining stabler and higher effectiveness.

When used as pharmaceuticals, cosmeceutical, cosmetics, foods such as functional foods, animal drugs, animal foods or animal feeds, the agent of the present invention can be prepared in the forms described below.

Useful dosage forms for pharmaceuticals include, but are not limited to, oral preparations (liquid preparations such as extracts, elixirs, syrups, tinctures, and lemonades; solid preparations such as capsules, granules, pills, powders, and tablets), injections, infusions, nasal drops, eye drops, suppositories, sprays, and dosage forms for percutaneous administration, such as ointments and patches.

Useful forms for cosmeceuticals include, but are not limited to, non-pharmaceutical preparations for oral ingestion, such as drinkable preparations, and non-pharmaceutical preparations for topical application, such as eye drops and nasal drops, and non-pharmaceutical preparations for skin application, such as fomentations and skin creams.

Useful forms for cosmetics include, but are not limited to, toilet water, creams, lotions, gels, mists, masks, packs, shampoos, and rinses.

Useful forms for foods include, but are not limited to, general foods such as food oil or fat compositions, cooking oils, spray oils, butters, margarines, shortenings, whipped creams, condensed milks, whiteners, dressings, pickling solutions, breads, cakes, pies, cookies, Japanese confectionery, snacks, fried confectionery, chocolates and chocolate confectionery, rice confectionery, roux, sauces, dips, toppings, ice creams, noodles, bakery mixes, fried foods, processed meats, fish paste products, frozen foods such as frozen entrees, frozen livestock product foods, and frozen agricultural product foods, cooked rice, jams, cheeses, cheese foods, cheese-like foods, gums, candies, fermented milks, canned foods, and beverages, eaten with the addition or blending of the agent of the present invention, as well as functional foods such as supplements, foods for specified health uses, health foods, and nutritional supplementary foods, orally taken for non-pharmaceutical purposes to maintain or promote health.

Useful dosage forms for animal drugs include oral preparations (liquid preparations such as extracts, elixirs, syrups, tinctures, and lemonades; solid preparations such as capsules, granules, pills, powders, and tablets), injections, nasal drops, eye drops, suppositories, and preparations for transdermal administration, such as ointments and patches.

Likely ways of using the agent of the present invention for animal feeds or foods include direct feeding thereof to animals, and feeding of an animal feed or food containing the agent. Furthermore, the agent of the present invention can be used as health foods and nutritional supplementary foods for animals in the same manner.

The agent of the present invention may incorporate any other active ingredients without limitations, as long as the formulation thereof with coenzyme Q does not produce unwanted interactions. Such other active ingredients include anticancer agents, as well as analgesics, antipruritics, antibiotics, hair growth promoters and the like. The dosage form of the agent of the present invention is not subject to limitation; for example, the dosage form may be a powder, may be granules with the addition of a binder, may be a powder coated with a coating agent (for example, the cellulose derivatives ethyl cellulose and hydroxypropylmethylcellulose phthalate, gum arabic, carmellose, synthetic silica, cetanol, vinyl acetate resin), and may be capsules filled with a powder, granules, or coated powder. The agent of the present invention may also be formulated with natural oils (e.g., olive oil, corn oil, palm oil, palm kernel oil, coconut oil, soybean oil, sesame oil, rapeseed oil, cottonseed oil, sunflower seed oil, safflower oil, almond oil, rice oil, camellia oil, perilla oil, linseed oil), oily higher fatty acids (e.g., palmitic acid, stearic acid, oleic acid, arachidonic acid), higher fatty acid monoglycerides (e.g., lauric acid monoglyceride, myristic acid monoglyceride, stearic acid monoglyceride, oleic acid monoglyceride), surfactants (e.g., glycerin fatty acid esters, sucrose fatty acid esters, sorbitan fatty acid esters, propylene glycol fatty acid esters) or a mixture thereof. While in the oily state, the agent of the present invention may be filled to obtain soft capsules. In this case, gelatin-based dosage forms or dosage forms based on other watersoluble polymeric substances can also be used. Such capsules include microcapsules.

Furthermore, the agent of the present invention may be prepared as emulsions (e.g., W/O emulsions, O/W emulsions) using surfactants, oils and fats, mineral oils (e.g., GP9) to obtain a liquid preparation.

It is desirable that the agent of the present invention be prepared in a dosage form showing an effect when taken orally. For skin cancer, however, a dosage form permitting direct application to the skin is desirable. In this regard, the dosage form is exemplified by, but not limited to, a dosage form prepared by dissolving or mixing and dispersing the agent of the present invention in an appropriate base (e.g., hydrophilic ointments, water-absorbing ointments, simple ointments, macrogol ointments, plastibase) to obtain a cream, paste, jelly, gel, emulsion or liquid form (such as ointments, liniments, lotions, sprays), as well as a dosage form prepared by dissolving or mixing and dispersing a reduced coenzyme Q in an adhesive, and extending the solution or dispersion over a support (plasters, tapes).

In preparing the agent of the present invention, the content of reduced coenzyme Q, dosage form, method of storage and storage style can be determined as appropriate according to intended use, such as pharmaceuticals, cosmeceuticals, cosmetics, foods, functional foods, animal drugs, or animal feeds. The content of reduced coenzyme Q in the agent of the present invention is preferably 0.001 to 99.9% by weight, more preferably 0.001 to 20% by weight, still more preferably 0.005 to 10% by weight.

### Examples

The present invention is hereinafter described in more detail by means of the following experimental examples and production examples, whichare illustrative embodiments according to the original disclosure, but do not illustrate embodiments of the invention as defined by the appended claims.

### <Production Example 1> Production of Reduced Coenzyme Q₁₀

100 g of oxidized coenzyme Q₁₀ (purity 99.4%) and 60 g of L-ascorbic acid were added to 1000 g of ethanol, and a reducing reaction was performed with stirring at 78°C. After 30 hours, the reaction mixture was cooled to 50°C, and while maintaining the temperature, 330 g of ethanol and 70 g of water were added. While stirring, this ethanol solution (containing 100 g of reduced coenzyme Q₁₀) was cooled to 2°C at a cooling rate of 10°C/hour to yield a white slurry. This slurry was filtered under reduced pressure; the resulting wet crystal was sequentially washed with cold ethanol, cold water, and cold ethanol in this order (the cold solvents for the washing were used at 2°C), and the wet crystal was dried under reduced pressure (20 to 40°C, 1 to 30 mmHg) to yield 97 g of a white dry crystal of reduced coenzyme Q₁₀. All operations but drying under reduced pressure were performed in a nitrogen atmosphere.

### <Production Example 2> Dissolution of Reduced Coenzyme Q₁₀ in LDL (low-density lipoprotein)

Human LDL (BIODESIGN) and Medium 106S (Kurabo Industries, Ltd.), a basal medium for normal human skin fibroblasts, were mixed to prepare a LDL-containing medium. The reduced coenzyme Q₁₀ obtained in Production Example 1 was dissolved into the LDL-containing medium at 50°C with sonication. This was followed by membrane filtration, and the filtrate obtained was used as the test sample stock solution.

Likewise, the LDL-containing medium as is (without adding reduced coenzyme Q₁₀) was subjected to membrane filtration and the filtrate obtained was used as the control sample stock solution. The LDL concentrations of the test sample stock solution and the control sample stock solution were same.

The concentration of reduced coenzyme Q₁₀ in the test sample stock solution was measured by high performance liquid chromatography (column: length 25 cm, diameter 4.6 mm, YMC-PACK ODS-A (YMC), mobile phase: methanol-hexane (85:15, v/v), detection wavelength: 290 nm, flow rate: 1.0 mL/min). As a result, the weight percent of reduced coenzyme Q₁₀ based on the total weight of coenzyme Q₁₀ in the test sample stock solution was calculated to be 84.1% by weight, and the concentration of reduced coenzyme Q₁₀ in the test sample stock solution was calculated to be 234.68 µg/ml. The test sample stock solution and control sample stock solution were subjected to the serial dilution with the basal medium Medium 106S to make diluted samples. The concentrations of the test sample stock solution or the control sample stock solution in the diluted samples were 1.04, 2.08, 4.17, 8.33, 16.7 and 33.3 % (the concentrations of the reduced coenzyme Q₁₀ in a test sample were 2.44, 4.89, 9.87, 19.6, 39.1 and 78.2 µg/ml, respectively). The diluted samples were used in the following Example 1 as test samples or control samples.

### <Example 1> Suppressive Effect of Reduced Coenzyme Q₁₀ on LDL-induced Cell Proliferation

Normal human skin fibroblasts (NHDF, Kurabo Industries) were cultured in a Medium 106S basal medium (Kurabo Industries) supplemented with 2% fetal bovine serum, 10 µg/ml heparin, 1 µg/ml hydrocortisone, 10 ng/ml human recombinant epithelial growth factor, and 3 ng/ml human recombinant basic fibroblast growth factor, at 37°C in the presence of 5% CO₂. After becoming confluent, the cells were sown to a 96-well microplate at 2.0x10³ cells per well. The medium used at the time of cell sowing was a Medium 106S (Kurabo Industries) supplemented with 2% fetal bovine serum, 10 µg/ml heparin, and 1 µg/ml hydrocortisone. After cultivation for 24 hours, the medium was replaced with the same medium but supplemented with a test sample as obtained in Production Example 2, and the cells were further cultured for 48 hours.

For control, an LDL-free medium and a medium supplemented with a control sample as obtained in Production Example 2 were used.

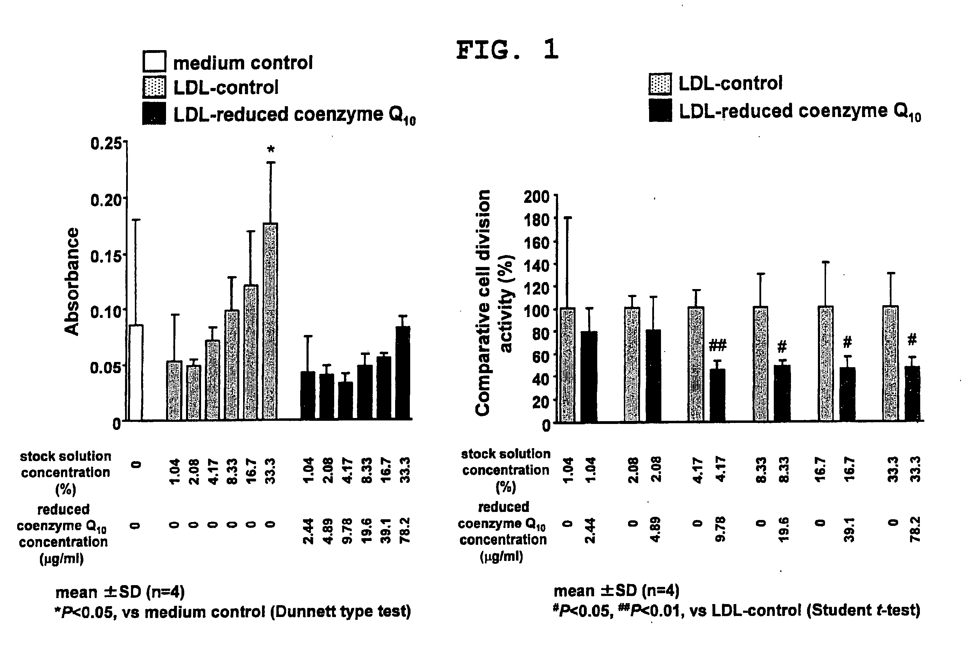

The medium was then removed from the 96-wel microplate, and the amount of BrdU (bromodeoxyuridine) incorporated in the nucleus was determined using an ELISA kit for cell proliferation in the presence of BrdU (Cell Proliferation BrdU, ELISA, Roche). Specifically, after the cells were labeled with BrdU, proteins were fixed and denatured, and an anti-BrdU antibody was added. The microplate was then washed, and a color developing reagent was added. The absorbance of the resulting blue color was determined at a wavelength of 405 nm. The results are shown in FIG. 1.

For statistical analysis, a parametric multiple comparison (Dunnett type) was performed versus a control group without sample addition; * indicates a significance level of less than 5%.

When LDL served as the control, Student's test was performed for each concentration of LDL; # indicates a significance level of less than 5%, and ## indicates a significance level of less than 1%.

When LDL alone was added (control sample), cell division activity increased dose dependently, compared with the medium control, as can be seen by the absorbance in Fig. 1. In the case of an LDL containing reduced coenzyme Q₁₀ (test sample), the elevation of cell division activity was suppressed compared with an LDL not containing reduced coenzyme Q₁₀ (control sample), as can be seen in Fig. 1 where the control sample is given a cell division activity value of 100% and the cell division activity values for the test samples are compared to this value. Hence, it was confirmed that reduced coenzyme Q₁₀ suppresses the abnormal cell proliferation induced by LDL in skin fibroblasts.

### <Example 2> Suppressive Effects of Oxidized Coenzyme Q₁₀ and Reduced Coenzyme Q₁₀ on Cell Activation in Cancer-Derived Cells

The reduced coenzyme Q₁₀ obtained in Production Example 1 and the oxidized coenzyme Q₁₀ used as a raw material in Production Example 1 were respectively dissolved in undiluted Tween 80 (MP Biomedicals) to give test samples.

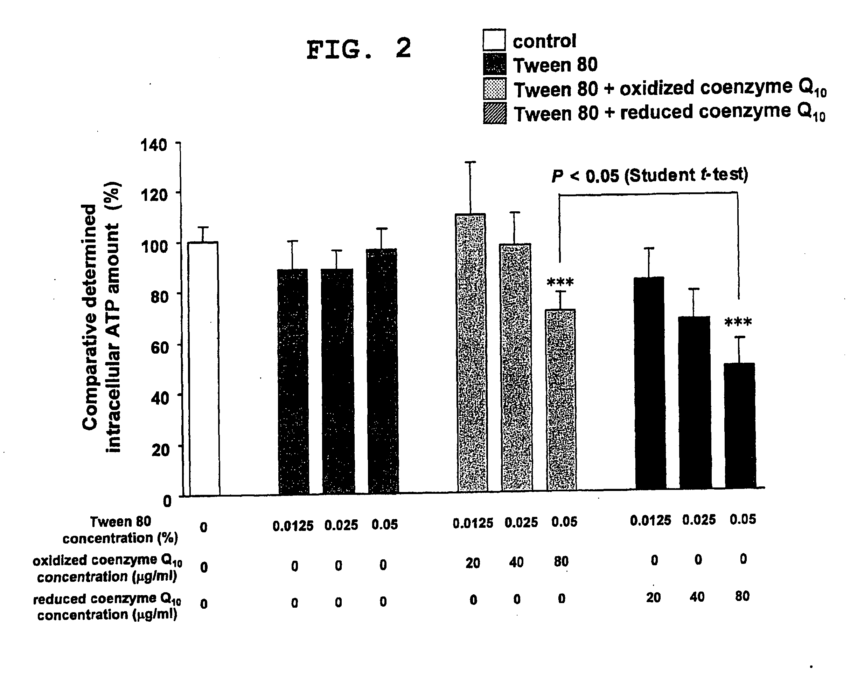

Human breast cancer-derived cells (MCF-7, supplied by ATCC) were cultured in a Dulbecco's modified MEM medium (GIBCO) supplemented with 200 U of penicillin, 0.1 µg/ml streptomycin, and 10% fetal bovine serum at 37°C in the presence of 5% CO₂. After becoming confluent, the cells were sown to a 96-well microplate (IWAKI) at 2.0x10⁴ cells per well. Twenty-four hours after the sowing, the medium was replaced with a Dulbecco's modified MEM medium containing 0.5% fetal bovine serum, and the above test samples were added to said medium such that the concentrations of Tween 80 were to be 0.0125, 0.025 and 0.05 % (the concentrations of the reduced coenzyme Q₁₀ or oxidized coenzyme Q₁₀ in the medium were 20, 40 and 80 µg/ml). Tween 80 alone was added to the Dulbecco's modified MEM medium containing 0.5% fetal bovine serum such that the concentrations were to be the same, and the resulting solution was used as a control. Twenty-four hours later, the medium was removed, and the microplate was washed with PBS (GIBCO), after which intracellular ATP contents were determined using an ATP assay kit (Perkin-Elmer) and compared. The results are shown in FIG. 2, where the determined intracellular ATP amount for the control sample is given a value of 100% and the determined intracellular ATP amounts for the test samples were compared to this value.

Compared with the control group not containing the test samples, the intracellular ATP content decreased significantly with the addition of 80 µg/ml oxidized coenzyme Q₁₀ and the addition of 80 µg/ml reduced coenzyme Q₁₀ (P<0.001). Comparing oxidized coenzyme Q₁₀ and reduced coenzyme Q₁₀, significantly lower intracellular ATP contents were obtained with the addition of 80 µg/ml reduced coenzyme Q₁₀ than with the addition of 80 µg/ml oxidized coenzyme Q₁₀ (P<0.05). These results confirm that both oxidized coenzyme R₁₀ and reduced coenzyme Q₁₀ have a "cancer therapeutic" effect by suppressing the activation of cancer-derived cells, and, quite surprisingly, that reduced coenzyme Q₁₀ is more effective than oxidized coenzyme Q₁₀ with a statistically significant difference.

### <Comparative Example 1> Promotive Effect of Reduced Coenzyme Q₁₀ on Cell Activation in Skin Fibroblasts

The reduced coenzyme Q₁₀ obtained in Production Example 1 and the oxidized coenzyme Q₁₀ used as a raw material in Production Example 1 were respectively dissolved in undiluted HCO-60 (polyoxyethylene hydrogenated castor oil, Nihon Surfactant Kogyo K.K.) to give test samples.

Normal human skin fibroblasts (NHDF, Kurabo Industries) were cultured in a Medium 106S basal medium (Kurabo Industries) supplemented with 2% fetal bovine serum, 10 µg/ml heparin, 1 µg/ml hydrocortisone, 10 ng/ml human recombinant epithelial growth factor, and human recombinant basic fibroblast growth factor 3 ng/ml, at 37°C in the presence of 5% CO₂. After becoming confluent, the cells were sown to a 96-well microplate at 2.0x10³ cells per well. The medium used at the time of cell sowing was a Medium 106S (Kurabo Industries, Ltd.) supplemented with 2% fetal bovine serum, 10 µg/ml heparin, and 1 µg/ml hydrocortisone. After cultivation for 24 hours, the test samples were replaced with the same medium added with the oxidized or reduced coenzyme Q₁₀ such that the concentrations were to be 0.12 and 0.98 µg/ml, and the cells were further cultured for 48 hours. The same amount of HCO-60 alone was added to the medium, and the resulting solution was used as a control. The medium was then removed, and the microplate was washed with PBS (GIBCO), after which intracellular ATP contents were determined using an ATP assay kit (Perkin-Elmer) and compared. The results are shown in Table 1.

For statistical analysis, a parametric multiple comparison (Tukey type) was performed. In comparison with the solvent-only control group, * indicates a significance level of less than 5%, and ** indicates a significance level of less than 1%. In comparison with oxidized coenzyme Q₁₀, t indicates a significance level of less than 5%.

Compared with the solvent control, significantly elevated intracellular ATP levels were observed with oxidized coenzyme Q₁₀ and with reduced coenzyme Q₁₀.

Comparing oxidized coenzyme Q₁₀ and reduced coenzyme Q₁₀, significantly elevated intracellular ATP levels were observed with reduced coenzyme Q₁₀. These results demonstrated that reduced coenzyme Q₁₀ didn't adversely affect activation of normal cells, but inhibited specifically that of cancer cells.

**Table 1**

| | Oxidized or reduced coenzyme Q₁₀ concentration (µg/ml) | |
|---|---|---|
| | 0.12 | 0.98 |
| | ATP level (nM) | |
| Solvent control | 146.37±16.41 | 141.99±42.56 |
| Oxidized coenzyme Q₁₀ | 183.23±62.70* | 224.84±43.55* |
| Reduced coenzyme Q₁₀ | 277.21±46.02**† | 267.24±20.72** |

| | | |
|---|---|---|
| **P* < 0.05, **P < 0.01, compared with solvent (Tukey test) † *P* < 0.05, compared with oxidized coenzyme Q₁₀ (Tukey test) | | |

### <Example 3> Suppressive Effect of Reduced Coenzyme Q₁₀ on Carcinogenesis in Rat Colorectal Carcinogenesis Model

To examine the effect of reduced coenzyme Q₁₀ on carcinogenesis, a test was performed using a rat colorectal carcinogenesis model. This test method represents a rodent model of two-stage carcinogenesis, targeting the rat large intestine. Because carcinogenesis research using this test method is widely conducted for screening for cancer prophylactic agents and other purposes, the test results are sufficiently reliable.

Twenty-seven male F344 rats at 6 weeks of age were divided into three groups (9 animals/group) by computer-based randomized block design in a way such that no statistically significant difference would be produced in mean body weight. For initiation treatment for colorectal carcinogenesis, each rat was given azoxymethane (AOM) by subcutaneous administration at a dose of 15 mg/kg body weight once weekly for two weeks.

Starting on the day before administration of AOM, reduced coenzyme Q₁₀ in solution in vehicle (olive oil) at a concentration of 20 or 50 mg/ml was given by gavage at doses of 0 (control group, group 1), 20, and 50 mg/kg (groups 2 and 3) on consecutive days for four weeks. The control group (group 1) received only the vehicle for administering reduced coenzyme Q₁₀.

Four weeks after initial administration of reduced coenzyme Q₁₀, all rats were autopsied, and the large intestine (colon and rectum, excluding the cecum) was extirpated. The large intestine with its ends ligated was fixed with injected 10% neutrally buffered formalin solution, after which the large intestine was incised and applied over filter paper flatly. Subsequently, the large intestine was post-fixed with 10% neutrally buffered formalin solution. After fixation, the large intestine was stained with 0.2% Methylene Blue solution in physiological saline for about 10 minutes, and the mucosal surface of the large intestine was examined using a stereomicroscope, whereby aberrant crypt foci (ACF), a form of precancerous lesion in colorectal cancer, were counted.

ACF was counted and classified by number of crypts present therein. Data were obtained as total ACF number per large intestine (colon and rectum) specimen, total crypt number per large intestine specimen, and mean crypt number per ACF (Table 2).

Furthermore, total ACF number per large intestine specimen was calculated according to the number of crypts in each ACF (Table 3).

For statistical analysis, Bartlett's homogeneity test was performed to compare group 1 and groups 2 and 3. When the dispersion was found to be homogenous, Dunnett's multiple comparison test based on a parametric approach was performed. In the case of non-homogenous dispersion, Dunnett's multiple comparison test based on a non-parametric approach (joint ranking method) was performed.

**Table 2**

| ACF Formation in the Rat Large Intestine | | | | | | |
|---|---|---|---|---|---|---|
| Group | Test substance | Dose (mg/ kg) | n | Total ACF number/ large intestine specimen | Total ACs number/ large intestine specimen | Mean ACs number/ focus |
| 1 | Olive oil (control group) | - | 9 | 142±26 (100) | 254±53 (100) | 1.79±0.12 |
| 2 | Reduced coenzyme Q₁₀ | 20 | 9 | 113±32 (80) | 214±81 (84) | 1.77±0.16 |
| 3 | Reduced coenzyme Q₁₀ | 50 | 9 | 104±26 (74)* | 181±54 (71)* | 1.76±0.33 |

| | | | | | | |
|---|---|---|---|---|---|---|
| ACF: Aberrant crypt foci ACs: Aberrant crypts Mean ± standard deviation *: A significant difference versus group 1 (P<0.05) Numerical figures in parentheses are percent values based on a 100 % value assigned to the value for the control group. | | | | | | |

**Table 3**

| Number of ACFs Formed in the Rat Large Intestine (distribution by crypt number) | | | | | | | |
|---|---|---|---|---|---|---|---|
| group | test substance | dose (mg/kg) | n | 1 crypt | 2 crypts | 3 crypts | 4 crypts or more |
| 1 | Olive oil (control group) | - | 9 | 66±14 (100) | 50±10 (100) | 18±5 (100) | 8±5 (100) |
| 2 | Reduced coenzyme Q₁₀ | 20 | 9 | 47±12 (71) ** | 39±9 (79) | 18±12 (100) | 7±6 (97) |
| 3 | Reduced coenzyme Q₁₀ | 50 | 9 | 40±10 (61) *** | 35±11 (70)** | 15±7 (80) | 6±4 (78) |

| | | | | | | | |
|---|---|---|---|---|---|---|---|
| Mean ± standard deviation **, ***: a significant difference versus group 1 (P<0.01, 0.001) Numerical figures in parentheses are percent values based on a 100 % value assigned to the value for the control group. | | | | | | | |

During the treatment period, no changes associated with administration of the test substance were observed in gross condition, mortality, food consumption, and body weight. Analyzing the data on ACF, a form of precancerous lesion in the large intestine, the total ACF number per large intestine specimen and the total crypt number per large intestine specimen decreased with administration of reduced coenzyme Q₁₀, compared with the control group. Particularly in the 50 mg/kg group, a statistically significant reduction was observed. In terms of total ACF number per large intestine specimen, calculated by crypt number in each ACF, the total ACF number for 1-crypt ACF or 2-crypt ACF decreased significantly with administration of reduced coenzyme Q₁₀.

These results show that reduced coenzyme Q₁₀ suppresses the onset of ACF, a form of precancerous lesion in colorectal cancer. Hence, this Example has demonstrated that reduced coenzyme Q₁₀ is effective in "carcinogenesis suppression" and/or "cancer prevention".

### <Preparation Example 1> (Powder)

Reduced coenzyme Q₁₀ was dissolved in propanol, and this solution was adsorbed to microcrystalline cellulose and then dried under reduced pressure. The resultant dried product was mixed with cornstarch to yield a powder. The weight amounts of the ingredients are shown below.

| | |
|---|---|
| Reduced coenzyme Q₁₀ | 9.5% by weight |
| Microcrystalline cellulose | 38.1% by weight |
| Cornstarch | 52.4% by weight |

### <Preparation Example 2> (Capsules)

A powder was prepared with the formula shown below in the same manner as Preparation Example 1, and it was filled in gelatin capsules by a conventional method. The weight amounts of the ingredients are shown below.

| | |
|---|---|
| Reduced coenzyme Q₁₀ | 20% by weight |
| Microcrystalline cellulose | 30% by weight |
| Cornstarch | 20% by weight |
| Lactose | 25% by weight |
| Magnesium stearate | 3% by weight |
| Polyvinylpyrrolidone | 2% by weight |

### <Preparation Example 3> (Soft Capsules)

Corn oil was heated to 50°C and reduced coenzyme Q₁₀ molten at the same temperature was added and dissolved thereto. This was packed in soft capsules by a conventional method. The weight amounts of the ingredients are shown below.

| | |
|---|---|
| Reduced coenzyme Q₁₀ | 12.5% by weight |
| Corn oil | 87.5% by weight |

### <Preparation Example 4> (Tablets)

Reduced coenzyme Q₁₀ was dissolved in propanol, and this solution was adsorbed to microcrystalline cellulose and then dried under reduced pressure. The resultant dried product was blended with cornstarch, lactose, carboxymethylcellulose, and magnesium stearate, an aqueous solution of polyvinylpyrrolidone was added as the binder, and granulation was performed by a conventional method. This was blended with talc as a lubricant, after which the blend was tableted to yield tablets.

| | |
|---|---|
| Reduced coenzyme Q₁₀ | 15.63% by weight |
| Cornstarch | 19.53% by weight |
| Lactose | 11.72% by weight |
| Carboxymethylcellulose | 7.81% by weight |
| Microcrystalline cellulose | 31.25% by weight |
| Polyvinylpyrrolidone | 3.91% by weight |
| Magnesium stearate | 2.34% by weight |
| Talc | 7.81% by weight |

### <Preparation Example 5> (Hydrophilic Ointment)

A hydrophilic ointment containing reduced coenzyme Q₁₀ was prepared with the following composition by a commonly known method.

| | |
|---|---|
| Hydrophilic ointment | 96% by weight |
| Reduced coenzyme Q₁₀ | 1% by weight |
| Ascorbyl stearate | 3% by weight |

### <Preparation Example 6> (W/O Emulsion)

A W/O emulsion comprising reduced coenzyme Q₁₀ was prepared with the following composition by a commonly known method.

| | |
|---|---|
| Polyoxyethylene glycerol sorbitan fatty acid ester | 3.60% by weight |
| Polyoxyethylene fatty acid ester | 1.40% by weight |
| Stearyl alcohol | 2.00% by weight |
| Mineral oil, GP9 | 20.00% by weight |
| Paraben mixture | as appropriate |
| Magnesium sulfate (MgSO₄·7H₂O) | 0.70% by weight |
| Reduced coenzyme Q₁₀ | 1.00% by weight |
| Calcium chloride (CaCl₂) | 0.85% by weight |

Add deionized water to make a total volume of 100.00% by weight.

### Industrial Applicability

According to the present invention, an agent for use in a method for mitigation of adverse reactions of anticancer agents, which can be used in foods and beverages, such as general foods, health foods (supplements, health aid foods, nutritional supplementary foods, nutrient-fortified foods, nutrient-adjusted foods, health beverages) and health or nutrition functional foods (foods for specified health uses, foods with nutrient function claims), or in pharmaceuticals, cosmeceuticals, cosmetics can be provided.

## Claims

1. An agent for use in a method for mitigating adverse reactions of anticancer agents, said agent comprising a reduced coenzyme Q represented by the formula (1): 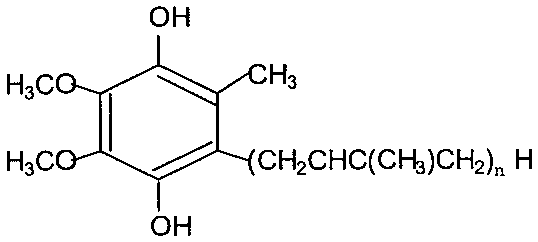 wherein n represents an integer of 1 to 12 as an active ingredient.

2. The agent for use in a method for mitigating adverse reactions of anticancer agents according to claim 1, wherein the reduced coenzyme Q is reduced coenzyme Q₁₀.

3. The agent for use in a method for mitigating adverse reactions of anticancer agents according to claim 1, comprising 0.001 to 99.9% by weight of the reduced coenzyme Q.

4. The agent for use in a method for mitigating adverse reactions of anticancer agents according to any one of claims 1 to 3, wherein the daily dose of the reduced coenzyme Q is 1 mg to 3000 mg.

5. The agent for use in a method for mitigating adverse reactions of anticancer agents according to any one of claims 1 to 4, which is a pharmaceutical, a cosmeceutical or a cosmetic.

6. The agent for use in a method for mitigating adverse reactions of anticancer agents according to any one of claims 1 to 4, which is a food or a beverage.

7. The agent for use in a method for mitigating adverse reactions of anticancer agents according to any one of claims 1 to 4, which is an animal drug, an animal feed or an animal food.

## Patentansprüche

1. Mittel zur Verwendung in einem Verfahren zur Minderung der Nebenwirkungen eines Antikrebsmittels, wobei das Mittel ein reduziertes Coenzym Q der Formel (1) : 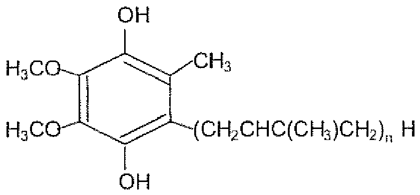 worin n eine ganze Zahl von 1 bis 12 darstellt, als Wirkstoff umfasst.

2. Mittel zur Verwendung in einem Verfahren zur Minderung der Nebenwirkungen eines Antikrebsmittels gemäss Anspruch 1, wobei das reduzierte Coenzym Q reduziertes Coenzym Q₁₀ ist.

3. Mittel zur Verwendung in einem Verfahren zur Minderung der Nebenwirkungen eines Antikrebsmittels gemäss Anspruch 1, umfassend 0,001 bis 99,9 Gew.% des reduzierten Coenzyms Q.

4. Mittel zur Verwendung in einem Verfahren zur Minderung der Nebenwirkungen eines Antikrebsmittels gemäss irgendeinem der Ansprüche 1 bis 3, wobei die tägliche Dosis an reduziertem Coenzym Q 1 bis 3.000 mg beträgt.

5. Mittel zur Verwendung in einem Verfahren zur Minderung der Nebenwirkungen eines Antikrebsmittels gemäss irgendeinem der Ansprüche 1 bis 4, das ein Arzneimittel, ein Kosmezeutikum oder eine Kosmetik ist.

6. Mittel zur Verwendung in einem Verfahren zur Minderung der Nebenwirkungen eines Antikrebsmittels gemäss irgendeinem der Ansprüche 1 bis 4, das ein Lebensmittel oder ein Getränk ist.

7. Mittel zur Verwendung in einem Verfahren zur Minderung der Nebenwirkungen eines Antikrebsmittels gemäss irgendeinem der Ansprüche 1 bis 4, das ein Tierarzneimittel, ein Futtermittel oder ein Tierfutter ist.

## Revendications

1. Agent pour une utilisation dans un procédé d'atténuation des réactions indésirables des agents anticancéreux, ledit agent comprenant un coenzyme Q réduit représenté par la formule (1) : 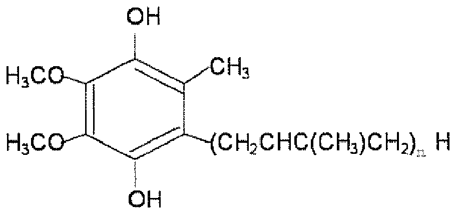 dans laquelle n représente un nombre entier de 1 à 12, en tant que principe actif.

2. Agent pour une utilisation dans un procédé d'atténuation des réactions indésirables des agents anticancéreux selon la revendication 1, où le coenzyme Q réduit est le coenzyme Q₁₀ réduit.

3. Agent pour une utilisation dans un procédé d'atténuation des réactions indésirables des agents anticancéreux selon la revendication 1, comprenant 0,001 à 99,9 % en poids de coenzyme Q réduit.

4. Agent pour une utilisation dans un procédé d'atténuation des réactions indésirables des agents anticancéreux selon l'une quelconque des revendications 1 à 3, où la dose quotidienne de coenzyme Q réduit est de 1 mg à 3000 mg.

5. Agent pour une utilisation dans un procédé d'atténuation des réactions indésirables des agents anticancéreux selon l'une quelconque des revendications 1 à 4, qui est un agent pharmaceutique, un agent cosméceutique ou un agent cosmétique.

6. Agent pour une utilisation dans un procédé d'atténuation des réactions indésirables des agents anticancéreux selon l'une quelconque des revendications 1 à 4, qui est un aliment ou une boisson.

7. Agent pour une utilisation dans un procédé d'atténuation des réactions indésirables des agents anticancéreux selon l'une quelconque des revendications 1 à 4, qui est un médicament pour animaux, un aliment pour le bétail ou les animaux domestiques.
